# NEUE EUROPÄISCHE PATENTSCHRIFT

(11) **EP 0 523 668 B2**
(45) Veröffentlichungstag und Bekanntmachung des Hinweises auf die Entscheidung über den Einspruch: **25.08.1999**
(45) Hinweis auf die Patenterteilung: 25.10.1995
(21) Anmeldenummer: 92112096.0
(22) Anmeldetag: 15.07.1992
(51) Int. Cl.: C07C 47/55, C07C 45/63

(54) **Verfahren zur Herstellung von Difluorbenzaldehyden**
Process for the preparation of difluorobenzaldehydes
Procédé pour la préparation de difluorobenzaldéhydes

(30) Priorität: 16.07.1991 DE 4123461
(43) Veröffentlichungstag der Anmeldung: 20.01.1993
(73) Patentinhaber: Clariant GmbH, 65929 Frankfurt am Main (DE)
(72) Erfinder: Papenfuhs, Theodor, Dr., W-6000 Frankfurt am Main 50 (DE); Kanschik-Conradsen, Andreas, Dr., W-6084 Gernsheim (Rhein) (DE)

(56) Entgegenhaltungen:
- EP-A- 0 032 077
- EP-A- 0 265 854
- EP-A- 0 289 942
- EP-A- 0 296 479
- CHEMISTRY LETTERS. Bd. 8, 1988, TOKYO JP Seiten 1355 - 1358 YASUO YOSHIDA ET AL. 'A Convenient Synthesis of Fluorobenzaldehydes by KF/ Ph4PBr/ 18-Crown-6 Reagent System'

## Beschreibung

Die Erfindung betrifft ein verbessertes Verfahren zur Herstellung von 2,4-und 2,6-Difluorbenzaldehyd in hohen Ausbeuten durch Umsetzung der entsprechenden Dichlorbenzaldehyde mit Alkalimetallfluoriden in einem dipolar aprotischen Lösungsmittel in Gegenwart eines Ethylenglycoldialkylethers. Difluorbenzaldehyde sind wichtige Vorprodukte zur Herstellung von Pharmazeutika und Pflanzenschutzmitteln.

Aus DE-PS 3 637 156 ist bekannt, daß man aus 2,4-Dichlorbenzaldehyd mit Kaliumfluorid in Sulfolan bei Temperaturen von 210-215 °C innerhalb von 15 h 2,4-Difluorbenzaldehyd in einer Ausbeute von 68 % der Theorie herstellen kann. Das Gewichtsmengenverhältnis von 2,4-Dichlorbenzaldehyd zu Sulfolan beträgt hierbei 1:5,7, was eine ungenügende Raum-Zeit-Ausbeute bedingt.

Ferner ist aus EP-PS 289 942 die Herstellung von 4-Fluorbenzaldehyd durch Umsetzung von 4-Chlorbenzaldehyd mit Kaliumfluorid unter Zusatz von Tetraphenylphosphoniumbromid und Tetraethylenglycoldimethylether in Abwesenheit eines Lösungsmittels in einer Ausbeute von 60 % der Theorie bekannt. Die Umsetzung in Abwesenheit von Tetraphenylphosphoniumbromid führt zu keiner Bildung von 4-Fluorbenzaldehyd. Der Nachteil dieses bekannten Verfahrens liegt in der Verwendung des kostspieligen Tetraphenylphosphoniumbromids.

Es bestand daher ein erhebliches Interesse für ein technisch günstigeres Verfahren zur Herstellung der genannten Difluorbenzaldehyde.

Es wurde nun gefunden, daß man Difluorbenzaldehyde der allgemeinen Formel (1) 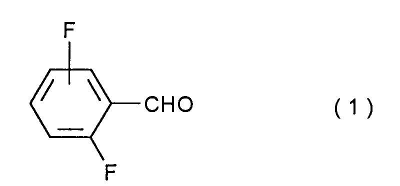 in welcher das zweite Fluoratom sich in 4- oder 6-Stellung befindet, überraschenderweise in hohen Ausbeuten durch Umsetzung eines Dichlorbenzaldehyds der allgemeinen Formel (2) 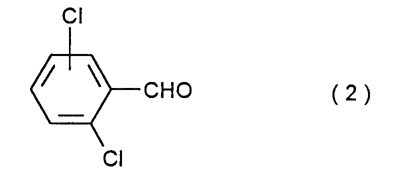 in welcher sich das zweite Chloratom in 4- oder 6-Stellung befindet, mit einem Alkalimetallfluorid in einem Lösungsmittel bei Temperaturen von etwa 160°C bis etwa 250°C, vorzugsweise von etwa 200°C bis etwa 230°C, vorteilhaft herstellen kann, wenn man die Umsetzung in einem dipolar aprotischen Lösungsmittel in Gegenwart eines Ethylenglykoldialkylethers als Katalysator der allgemeinen Formel (3)

RO-(CH₂-CH₂-O)ₙ-R (3)

in welcher R eine Methyl-, Ethyl- oder Propyl- bzw. Isopropyl-Gruppe und n eine Zahl von 1 bis etwa 50 bedeuten, und in Abwesenheit eines quartären Phosphoniumsalzes oder quartären Ammoniumsalzes durchführt.

An einzelnen Ethylenglycoldialkyl(C₁-c₃)-ethern seien beispielsweise genannt: Tetraethylenglycoldimethylether, Polyethylenglycoldimethylether 250, Polyethylenglycoldimethylether 500, Polyethylenglycoldimethylether 1000 und Polyethylenglycoldimethylether 2000. (Die angegebenen Zahlen bedeuten das mittlere Molekulargewicht.)

Als Alkalimetallfluoride kommen Natrium-, Kalium-, Rubidium- oder Cäsiumfluorid oder Kombinationen davon in Frage. Besonders geeignet sind Kaliumfluorid, Rubidiumfluorid oder Cäsiumfluorid oder Kombinationen davon.

Geeignete dipolar-aprotische Lösungsmittel sind beispielsweise Diphenylsulfon, Tetramethylensulfon, Dimethylsulfoxid, Tetramethylensulfoxid, Dimethylacetamid, Dimethylformamid oder N-Methylpyrrolidon oder Mischungen davon.

Als Ausgangsverbindungen der genannten allgemeinen Formel (2) dienen 2,4-oder 2,6-Dichlorbenzaldehyd.

Was die Mengenverhältnisse anbelangt, so ist es zweckmäßig, 1 Mol 2,4-bzw. 2,6-Dichlorbenzaldehyd mit etwa 0,8 bis etwa 1,6 Mol Alkalimetallfluorid, vorzugsweise etwa1,0 Mol bis etwa 1,4 Mol zur Umsetzung zu bringen.

Der als Katalysator dienende Ethylenglycoldialkylether wird zweckmäßigerweise in einer Menge von etwa 10 bis etwa 50 Gramm, bezogen auf 1 Mol eingesetzten Dichlorbenzaldehyd, angewendet.

Wichtig für die Verfahrensdurchführung ist, daß während der gesamten Reaktion eine gute Durchmischung der Reaktionssuspension erfolgt.

Das erfindungsgemäße Verfahren wird in der Regel bei Atmosphärendruck durchgeführt. Es kann jedoch auch bei Über- oder Unterdruck vorgenommen werden. In Abhängigkeit des Siedepunkts des angewandten dipolar aprotischen Lösungsmittels kann ein Arbeiten bei Überdruck angezeigt sein.

Die nachstehenden Beispiele dienen zur Erläuterung der Erfindung, ohne sie darauf zu beschränken.

### Beispiel 1

334 g (5,76 Mol) Kaliumfluorid werden in 960 g Sulfolan suspendiert. Der Suspension werden 420 g (2,4 Mol) 2,4-Dichlorbenzaldehyd und 60 g (0,3 Mol) Tetraethylenglycoldimethylether zugesetzt. Anschließend wird 13 h unter Stickstoff und heftigem Rühren auf 220 °C erhitzt. Dann werden aus der Reaktionssuspension alle bis 220 °C/13.3 mbar (10 Torr) flüchtigen Bestandteile abdestilliert, wodurch man 312,4 g eines Rohdestillates, das nach gaschromatographischer Analyse 254,6 g (74,7 % d. Th.) 2,4-Difluorbenzaldehyd und 36,2 g (9,5 % d. Th.) Chlorfluorbenzaldehyd enthält, erhält.

Durch anschließende Fraktionierung erhält man 245,9 g (72,2 % d. Th.) 2,4-Difluorbenzaldehyd.

Führt man die Reaktion in 1120 g Diphenylsulfon statt in 960 g Sulfolan durch und verfährt im übrigen wie in Beispiel 1 beschrieben, so gelangt man praktisch zum gleichen Ergebnis.

### Beispiel 2

Das Verfahren von Beispiel 1 wurde mit 60 g (0,24 Mol) Polyethylenglycoldimethylether 250 anstelle von Tetraethylenglycoldimethylether durchgeführt. Hierbei wurden 388,7 g eines Rohdestillats, das nach gaschromatographischer Analyse 252,6 g (74 % d. Th.) 2,4-Difluornitrobenzol und 33,4 g (8,8 % d. Th.) Chlorfluorbenzaldehyd enthält, erhalten.

Arbeitet man in 1120 g Diphenylsulfon statt in 960 g Sulfolan und verfährt im übrigen wie angegeben, so gelangt man praktisch zum gleichen Ergebnis.

### Beispiel 3

Das Verfahren von Beispiel 1 wurde mit 60 g (0,06 Mol) Polyethylenglycoldimethylether 1000 anstelle von Tetraethylenglycoldimethylether durchgeführt. Es wurden 314,9 g eines Rohdestillates, das nach gaschromatographischer Analyse 240,3 g (70,5 % d. Th.) 2,4-Difluorbenzaldehyd und 19,8 g (5,2 % d. Th.) Chlorfluorbenzaldehyd enthielt, erhalten.

## Patentansprüche

1. Verfahren zur Herstellung von Difluorbenzaldehyden der allgemeinen Formel (1) 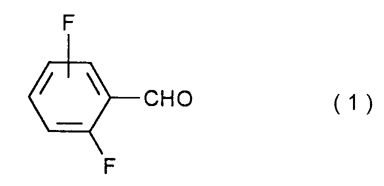 in welcher das zweite Fluoratom sich in 4- oder 6-Stellung befindet, durch Umsetzung eines Dichlorbenzaldehyds der allgemeinen Formel (2) 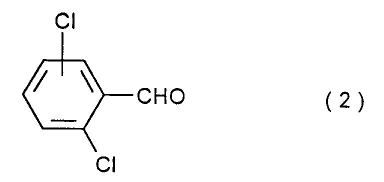 in welcher sich das zweite Chloratom in 4- oder 6-Stellung befindet, mit einem Alkalimetallfluorid in einem Lösungsmittel bei Temperaturen von 160°C bis 250°C, dadurch gekennzeichnet, daß man die Umsetzung in einem dipolar aprotischen Lösungsmittel in Gegenwart eines Ethylenglykoldialkylethers als Katalysator der allgemeinen Formel (3)
RO-(CH₂-CH₂-O)ₙ-R (3)
in welcher R eine Methyl-, Ethyl- oder Propyl- bzw. Isopropyl-Gruppe und n eine Zahl von 1 bis 50 bedeuten, und in Abwesenheit eines quartären Phosphoniumsalzes oder quartären Ammoniumsalzes durchführt.

2. Verfahren nach Anspruch 1, dadurch gekennzeichnet, daß man bei Temperaturen von 200 °C bis 230 °C umsetzt.

3. Verfahren nach mindestens einem der Ansprüche 1 und 2, dadurch gekennzeichnet, daß man mit Natrium-, Kalium-, Rubidium- oder Cäsiumfluorid oder Kombinationen davon umsetzt.

4. Verfahren nach mindestens einem der Ansprüche 1 bis 3, dadurch gekennzeichnet, daß man mit Kalium-, Rubidium- oder Cäsiumfluorid oder Kombinationen davon umsetzt.

5. Verfahren nach mindestens einem der Ansprüche 1 bis 4, dadurch gekennzeichnet, daß man als dipolar aprotisches Lösungsmittel Tetramethylensulfon, Dimethylsulfoxid, Tetramethylensulfoxid, Diphenylsulfon, Dimethylacetamid, Dimethylformamid oder N-Methylpyrrolidon oder Mischungen davon verwendet.

6. Verfahren nach mindestens einem der Ansprüche 1 bis 5, dadurch gekennzeichnet, daß man in Gegenwart von Tetramethylenglycoldimethylether, Polyethylenglycoldimethylether 250, Poylethylenglycoldimethylether 500, Polyethylenglycoldimethylether 1000 oder Polyethylenglycoldimethylether 2000 als Katalysator umsetzt, wobei die Zahlen das mittlere Molekulargewicht bedeuten.

7. Verfahren nach mindestens einem der Ansprüche 1 bis 6, dadurch gekennzeichnet, daß man die Reaktionssuspension während der gesamten Reaktion gut durchmischt.

8. Verfahren nach mindestens einem der Ansprüche 1 bis 7, dadurch gekennzeichnet, daß man bei Atmosphärendruck, Über- oder Unterdruck arbeitet.

## Claims

1. A process for the preparation of a difluorobenzaldehyde of the formula (1) 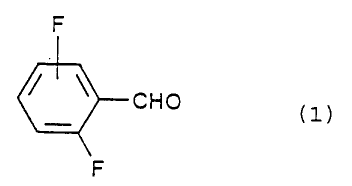 in which the second fluorine atom is in the 4 or 6 position, by reacting a dichlorobenzaldehyde of the formula (2) 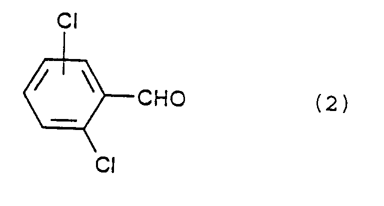 in which the second chlorine atom is in the 4 or 6 position, with an alkali metal fluoride in a solvent at temperatures of 160°C to 250°C, wherein the reaction is carried out in a dipolar aprotic solvent in the presence of an ethylene glycol dialkyl ether as catalyst of the formula (3)
RO-(CH₂-CH₂-O)ₙ-R (3)
in which R is a methyl, ethyl or propyl or isopropyl group and n is a number from 1 to 50, and in the absence of quaternary phosphonium salt or quaternary ammonium salt.

2. The process as claimed in claim 1, wherein the reaction is carried out at temperatures of 200°C to 230°C.

3. The process as claimed in at least one of claims 1 and 2, wherein the reaction is carried out using sodium fluoride, potassium fluoride, rubidium fluoride or cesium fluoride or combinations thereof.

4. The process as claimed in at least one of claims 1 to 3, wherein the reaction is carried out using potassium fluoride, rubidium fluoride or cesium fluoride or combinations thereof.

5. The process as claimed in at least one of claims 1 to 4, wherein the dipolar aprotic solvent used is tetramethylene sulfone, dimethyl sulfoxide, tetramethylene sulfoxide, diphenyl sulfone, dimethylacetamide, dimethylformamide or N-methylpyrrolidone or mixtures thereof.

6. The process as claimed in at least one of claims 1 to 5, wherein the reaction is carried out in the presence of tetramethylene glycol dimethyl ether, polyethylene glycol dimethyl ether 250, polyethylene glycol dimethyl ether 500, polyethylene glycol dimethyl ether 1000 or polyethylene glycol dimethyl ether 2000 as catalyst, the numbers denoting the mean molecular weight.

7. The process as claimed in at least one of claims 1 to 6, wherein the reaction suspension is thoroughly mixed during the entire reaction.

8. The process as claimed in at least one of claims 1 to 7, wherein atmospheric pressure, overpressure or underpressure is employed.

## Revendications

1. Procédé pour la préparation de difluorobenzaldéhydes de formule générale (1) : 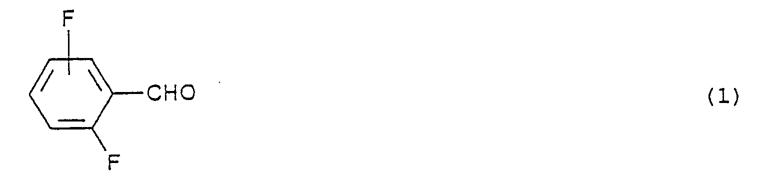 dans laquelle le deuxième atome de fluor se trouve en position 4 ou 6, par réaction d'un dichlorobenzaldéhyde de formule générale (2) : 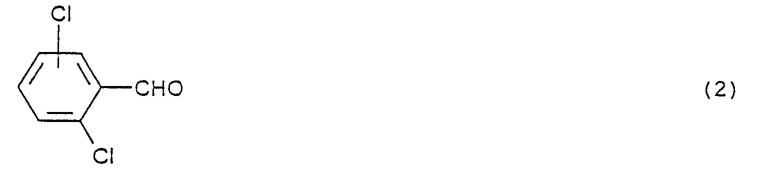 dans laquelle le deuxième atome de chlore se trouve en position 4 ou 6, avec un fluorure de métal alcalin dans un solvant à des températures de 160°C à 250°C, caractérisé en ce qu'on effectue la réaction dans un solvant aprotique dipolaire en présence d'un éther dialkylique de l'éthylèneglycol comme catalyseur de formule générale (3) :
RO-(CH₂-CH₂-O)ₙ-R (3)
dans laquelle R représente un groupe méthyle, éthyle ou propyle, respectivement isopropyle, et n est un nombre de 1 à 50, et en l'absence d'un sel de phosphonium quaternaire ou d'un sel d'ammonium quaternaire.

2. Procédé selon la revendication 1, caractérisé en ce qu'on effectue la réaction à des températures de 200°C à 230°C.

3. Procédé selon au moins l'une des revendications 1 et 2, caractérisé en ce qu'on effectue la réaction avec du fluorure de sodium, de potassium, de rubidium ou de césium ou leurs combinaisons.

4. Procédé selon au moins l'une des revendications 1 à 3, caractérisé en ce qu'on effectue la réaction avec le fluorure de potassium, de rubidium ou de césium ou leurs combinaisons.

5. Procédé selon au moins l'une des revendications 1 à 4, caractérisé en ce qu'on utilise comme solvants aprotiques dipolaires la tétraméthylènesulfone, le diméthylsulfoxyde, le tétraméthylènesulfoxyde, la diphénylsulfone, le diméthylacétamide, le diméthylformamide ou la N-méthylpyrrolidone ou leurs mélanges.

6. Procédé selon au moins l'une des revendications 1 à 5, caractérisé en ce qu'on fait réagir en présence d'éther diméthylique du tétraméthylèneglycol, d'éther diméthylique du polyéthylèneglycol 250, d'éther diméthylique du polyéthylèneglycol 500, d'éther diméthylique du polyéthylèneglycol 1000 ou d'éther diméthylique du polyéthylèneglycol 2000, en tant que catalyseur, les chiffres signifiant le poids moléculaire moyen.

7. Procédé selon au moins l'une des revendications 1 à 6, caractérisé en ce qu'on effectue un brassage intime de la suspension réactionnelle pendant la totalité de la réaction.

8. Procédé selon au moins l'une des revendications 1 à 7, caractérisé en ce qu'on travaille à la pression atmosphérique, à des pressions supérieures ou inférieures.
